# EUROPEAN PATENT SPECIFICATION

(11) **EP 2 467 500 B1**
(45) Date of publication and mention of the grant of the patent: **01.06.2016**
(21) Application number: 10810539.6
(22) Date of filing: 18.08.2010
(51) Int. Cl.: C12N 9/02, C12N 15/82

(54) **DETECTION OF AAD-1 EVENT DAS-40278-9**
ERKENNUNG DES AAD-1-EREIGNISSES DAS-40278-9
DÉTECTION D'ÉVÉNEMENT AAD-1 DAS-40278-9

(30) Priority: 19.08.2009 US 235248 P; 23.04.2010 US 327366 P
(43) Date of publication of application: 27.06.2012
(73) Proprietor: Dow AgroSciences, LLC, Indianapolis, Indiana 46268 (US)
(72) Inventor: CUI, Yunxing, Cory, Carmel, IN 46032 (US); GREENE, Thomas, William, Zionsville, IN 46077 (US); NOVAK, Stephen, Westfield, IN 46074 (US); ZHOU, Ning, Zionsville, IN 46077 (US)
(74) Representative: Zwicker, Jörk
(86) International application number: PCT/US2010/045871
(87) International publication number: WO 2011/022471

(56) References cited:
- WO-A2-2005/059103
- WO-A2-2005/107437
- WO-A2-2007/053482
- WO-A2-2008/143993
- US-A1- 2002 073 448
- US-A1- 2007 089 201
- US-A1- 2009 093 366
- BUH GAAA PARIÄ METI ET AL: "Comparison of different real-time PCR chemistries and their suitability for detection and quantification of genetically modified organisms", BMC BIOTECHNOLOGY, BIOMED CENTRAL LTD. LONDON, GB, vol. 8, no. 1, 6 March 2008 (2008-03-06), page 26, XP021035696, ISSN: 1472-6750
- SUBHASH CHANDER ET AL: "Genetic dissection of tocopherol content and composition in maize grain using quantitative trait loci analysis and the candidate gene approach", MOLECULAR BREEDING, KLUWER ACADEMIC PUBLISHERS, DO, vol. 22, no. 3, 12 April 2008 (2008-04-12) , pages 353-365, XP019611834, ISSN: 1572-9788
- ZHANG J ET AL: "Mapping quantitative trait loci for oil, starch, and protein concentrations in grain with high-oil maize by SSR markers", EUPHYTICA, KLUWER ACADEMIC PUBLISHERS, DO, vol. 162, no. 3, 3 August 2007 (2007-08-03), pages 335-344, XP019603818, ISSN: 1573-5060
- ANTONIUS J.M. MATZKE ET AL: "Position effects and epigenetic silencing of plant transgenes", CURRENT OPINION IN PLANT BIOLOGY, vol. 1, no. 2, 1 April 1998 (1998-04-01), pages 142-148, XP055048846, ISSN: 1369-5266, DOI: 10.1016/S1369-5266(98)80016-2
- GERMAN ET AL.: 'A rapid method for the analysis of zygosity in transgenic plants.' PLANT SCIENCE vol. 164, no. 2, February 2003, pages 183 - 187, XP008154095
- YANG ET AL.: 'tvent Specific Qualitative and Quantitative Polymerase Chain Reaction Detection of Genetically Modified MON863 Maize Based on the 5'-Transgene Integration Sequence.' J AGRIC FOOD CHEM vol. 53, no. 24, 30 November 2005, pages 9312 - 9318, XP008154145
- BRODMANN ET AL.: 'Real-Time Quantitative Polymerase Chain Reaction Methods for Four Genetically Modified Maize Varieties and Maize DNA Content in Food.' JOURNAL OF AOAC INTERNATIONAL vol. 85, no. 3, May 2002, pages 646 - 653, XP008154098
- SYLVAIN ET AL.: 'Rapid screening for HLA-B27 by a TaqMan-PCR assay using sequence- specific primers and a minor groove binder probe, a novel type of TaqMan probe.' JOURNAL OF IMMUNOLOGICAL METHODS vol. 287, no. 1-2, April 2004, pages 179 - 186, XP008154147
- GOTSCH ET AL.: 'Nuclease-Resistant Single-Stranded DNA Controls for Nucleic Acid Amplification Assays.' J CLIN MICROBIOL vol. 45, no. 8, August 2007, pages 2570 - 2574, XP008154099
- 'ABI PRISM 7000 Sequence Detection System and Applied Biosystems 7500 Real-Time PCR System.' APPLIED BIOSYSTEMS, [Online] 2007, page 1, XP008154148 Retrieved from the Internet: <URL:http://www3.appliedbiosystems.com/cms/ groups/applied markets supportldocuments/general d ocuments/cms 039287.pdf> [retrieved on 2010-10-08]
- DATABASE GENBANK 16 December 1995 XP008154574 Database accession no. ZMU16123
- DATABASE GENBANK 22 November 2002 XP008154588 Database accession no. AC133634
- DATABASE GENBANK 17 May 2006 XP008154566 Database accession no. DV681892

## Description

### BACKGROUND OF THE INVENTION

The *aad*-1 gene (originally from *Sphingobium herbicidovorans*) encodes the aryloxyalkanoate dioxygenase (AAD-1) protein. The trait confers tolerance to 2,4-dichlorophenoxyacetic acid and aryloxyphenoxypropionate (commonly referred to as "fop" herbicides such as diclofop and quizalofop) herbicides and may be used as a selectable marker during plant transformation and in breeding nurseries. The *aad-1* gene, itself, for herbicide tolerance in plants was first disclosed in WO 2005/107437 (*see also* US 2009-0093366).

Various methods of event detection are known. However, they each have issues. One method is the Pyrosequencing technique as described by Winge (Innov. Pharma. Tech. 00:18-24, 2000). In this method an oligonucleotide is designed that overlaps the adjacent genomic DNA and insert DNA junction. The oligonucleotide is hybridized to single-stranded PCR product from the region of interest (one primer in the inserted sequence and one in the flanking genomic sequence) and incubated in the presence of a DNA polymerase, ATP, sulfurylase, luciferase, apyrase, adenosine 5' phosphosulfate and luciferin. DNTPs are added individually and the incorporation results in a light signal that is measured. A light signal indicates the presence of the transgene insert/flanking sequence due to successful amplification, hybridization, and single or multi-base extension.

Fluorescence Polarization is another method that can be used to detect an amplicon of the present invention. Following this method, an oligonucleotide is designed which overlaps the genomic flanking and inserted DNA junction. The oligonucleotide is hybridized to single-stranded PCR product from the region of interest (one primer in the inserted DNA and one in the flanking genomic DNA sequence) and incubated in the presence of a DNA polymerase and a fluorescent-labeled ddNTP. Single base extension results in incorporation of the ddNTP. Incorporation can be measured as a change in polarization using a fluorometer. A change in polarization indicates the presence of the transgene insert/flanking sequence due to successful amplification, hybridization, and single base extension.

Molecular Beacons have been described for use in sequence detection. Briefly, a FRET oligonucleotide probe is designed that overlaps the flanking genomic and insert DNA junction. The unique structure of the FRET probe results in it containing secondary structure that keeps the fluorescent and quenching moieties in close proximity. The FRET probe and PCR primers (one primer in the insert DNA sequence and one in the flanking genomic sequence) are cycled in the presence of a thermostable polymerase and dNTPs. Following successful PCR amplification, hybridization of the FRET probe to the target sequence results in the removal of the probe secondary structure and spatial separation of the fluorescent and quenching moieties. A fluorescent signal results. A fluorescent signal indicates the presence of the flanking genomic/transgene insert sequence due to successful amplification and hybridization.

Hydrolysis probe assay, otherwise known as TAQMAN (PE Applied Biosystems, Foster City, Calif.), is a method of detecting and quantifying the presence of a DNA sequence. Briefly, a FRET oligonucleotide probe is designed that overlaps the genomic flanking and insert DNA junction. The FRET probe and PCR primers (one primer in the insert DNA sequence and one in the flanking genomic sequence) are cycled in the presence of a thermostable polymerase and dNTPs. During specific amplification, Taq DNA polymerase cleans and releases the fluorescent moiety away from the quenching moiety on the FRET probe. A fluorescent signal indicates the presence of the flanking/transgene insert sequence due to successful amplification and hybridization.

Another challenge, among many, is finding a suitable reference gene for a given test. For example, as stated in the abstract of Czechowski *et al.,* "An exceptionally large set of data from Affymetrix ATH1 whole-genome GeneChip studies provided the means to identify a new generation of reference genes with very stable expression levels in the model plant species Arabidopsis (*Arabidopsis thaliana*)*.* Hundreds of Arabidopsis genes were found that outperform traditional reference genes in terms of expression stability throughout development and under a range of environmental conditions." (Czechowski et al. (2005) Genome-wide identification and testing of superior reference genes for transcript normalization in Arabidopsis. Plant Physiol. 139, 5-17.)

Brodmann *et al.* (2002) relates to real-time quantitative PCR detection of transgenic maize content in food for four different maize varieties approved in the European Union. Brodmann, P.D., P.D., Ilg E.C., Berthoud H., and Herrmann, A. Real-Time Quantitative Polymerase Chain Reaction Methods for Four Genetically Modified Maize Varieties and Maize DNA Content in Food. J. of AOAC international 2002 85 (3)

Hernandez *et al.* (2004) mentions four possible genes for use with real-time PCR. Hernandez, M., Duplan, M.-N., Berthier, G., Vaitilingom, M., Hauser, W., Freyer, R., Pla, M., and Bertheau, Y. Development and comparison of four real-time polymerase chain reaction systems for specific detection and quantification of Zea mays L. J. Agric. Food Chem. 2004, 52, 4632-4637.

Costa *et al.* (2007) looked at these four genes (also in the real-time PCR context) and concluded that the alcohol dehydrogenase and zein genes were the best reference genes for detecting a sample "event" (a lectin gene) for transgenic feed intermix issues. Costa, L. D., and Martinelli L. Development of a Real-Time PCR Method Based on Duplo Target Plasmids for Determining an Unexpected Genetically Modified Soybean Intermix with Feed Components. J. Agric. Food Chem. 2007, 55, 1264-1273.

Huang *et al.* (2004) used plasmid pMulM2 as reference molecules for detection of MON810 and NK603 transgenes in maize. Huang and Pan, "Detection of Genetically Modified Maize MON810 and NK603 by Multiplex and Real-Time Polymerase Chain Reaction Methods," J. Agric. Food Chem., 2004, 52 (11), pp 3264-3268.

Gasparic *et al.* (2008) suggest LNA technology, from a comparison to cycling probe technology, TaqMan, and various real-time PCR chemistries, for quantitatively analyzing maize events (such as MON810). Ga 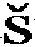 pari 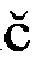 ,Cankar, 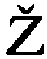 el, and Gruden, "Comparison of different real-time PCR chemistries and their suitability for detection and quantification of genetically modified organisms," BMC Biotechnol. 2008; 8: 26.

US 20070148646 relates to a primer extension method for quantification that requires controlled dispensation of individual nucleotides that can be detected and quantified by the amount of nucleotides incorporated. This is different from the TaqMan PCR method using an internal reference gene.

To distinguish between homozygous and hemizygous genotypes of TC1507, an Invader assay has been successfully used for this event. Gupta, M., Nirunsuksiri, W., Schulenberg, G., Hartl, T., Novak, S., Bryan, J., Vanopdorp, N., Bing, J. and Thompson, S. A non-PCR-based Invader Assay Quantitatively Detects Single-Copy Genes in Complex Plant Genomes. Mol. Breeding 2008, 21, 173-181.

Huabang (2009) relates to PCR-based zygosity testing of transgenic maize. However, no reference gene appears to be used. Huabang, "An Accurate and Rapid PCR-Based Zygosity Testing Method for Genetically Modified Maize," Molecular Plant Breeding, 2009, Vol.7, No.3, 619-623.

### BRIEF SUMMARY OF THE INVENTION

The subject invention provides assays for detecting the presence of the AAD-1 corn event designated DAS-40278-9 (having seed deposited with American Type Culture Collection (ATCC) with Accession No. PTA-10244) in a sample (of corn grain, for example). Kits and conditions useful in conducting the assays are also provided.

More specifically, the present invention relates in part to endpoint TaqMan PCR assays for the AAD-1 event in corn utilizing the maize endogenous reference gene invertase. Some embodiments are directed to assays that are capable of high throughput zygosity analysis. The subject invention further relates, in part, to the discovery of a preferred invertase reference gene for use in determining zygosity.

Thus, this invention also relates in part to plant breeding incorporating any of the subject detection methods. In some embodiments, said event can be "stacked" with other traits, including, for example, other herbicide tolerance gene(s) and/or insect-inhibitory proteins. The subject procedures can be used to uniquely identify corn lines comprising the event of the subject invention.

### BRIEF DESCRIPTION OF THE FIGURES

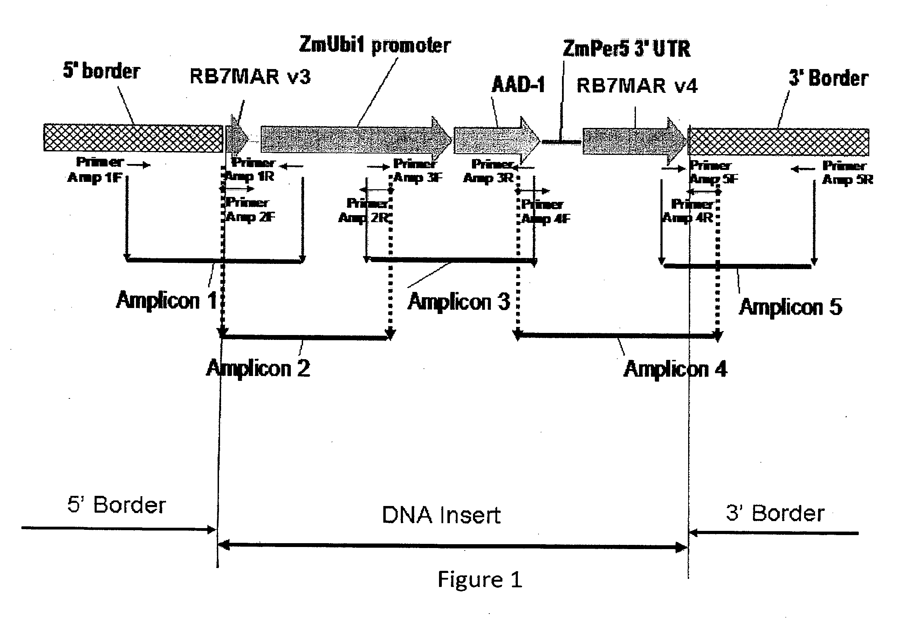

Figure 1 shows a cloning Strategy for the DNA Insert in the Corn Event DAS-40278-9.

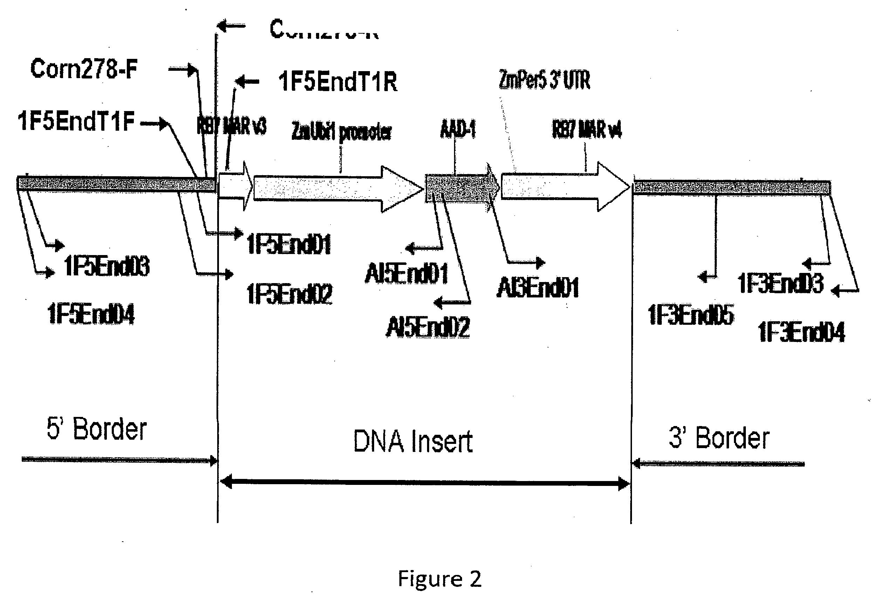

Figure 2 is a diagram of the Primers Used in PCR Amplification for Confirmation of Flanking Border Regions of the Corn Event DAS-40278-9 The schematic diagram depicts the Figure 2 is a diagram of the Primers Used in PCR Amplification for Confirmation of Flanking Border Regions of the Corn Event DAS-40278-9 The schematic diagram depicts the primer locations for confirming the full length sequencing of the AAD-1 corn event DAS-40278-9 from 5' to 3' borders.

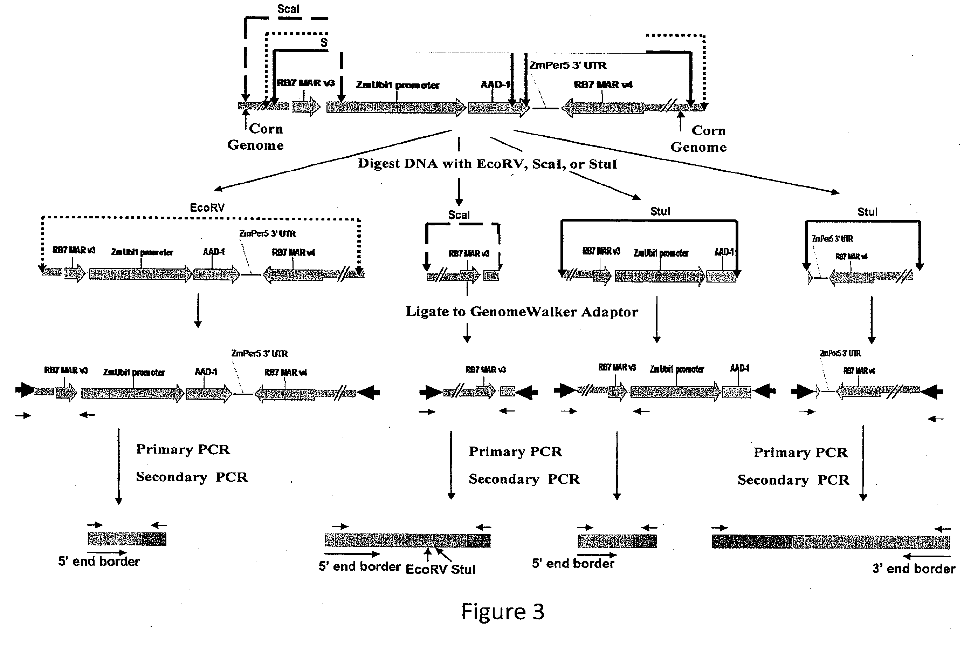

Figure 3 shows a cloning Strategy for the Flanking Border Sequences from the Corn Event DAS-40278-9 Genomic DNA of the Corn Event DAS-40278-9 was digested with *Eco*R V, *Stu* I, or *Sca* I and generated corresponding GenomeWalker™ libraries, which were used as templates to amplify the target DNA sequences.

### BRIEF DESCRIPTION OF THE SEQUENCES

SEQ ID NO:1 provides a sequence of 5' and 3' genomic flanking sequences on either side of the AAD-1 insert, including the insert, for Corn Event DAS-40278-9.
SEQ ID NOs: 2-7 are primers and probes for use according to the subject invention.
SEQ ID NO:8 is the exemplified event amplicon.
SEQ ID NO:9 is the exemplified reference amplicon.

### DETAILED DESCRIPTION OF THE INVENTION

Transgenic AAD-1 (providing herbicide tolerance) corn event DAS-40278-9 was generated by Whisker-mediated transformation. Both 5' and 3' end flanking sequences of this AAD-1 transgene insert were cloned, sequenced, and characterized as detailed inUSSN 61/235,248 (filed on August 19, 2009).

Specific TAQMAN primers and probe were designed, as detailed herein, in part according to the DNA sequences located in the 5' insert-to-plant junction. Event specificity of the primers and probe was successfully tested in duplex format with the corn invertase as a reference gene in real time PCR against 16 different AAD-1 corn events and two non-transgenic corn varieties. Procedures for end-point event specific TAQMAN assays for AAD-1 corn DAS-40278-9 were developed, as detailed herein.

The subject application discloses assays for detecting the presence of the subject transgenic corn event DAS-40278-9 (also known as pDAS1740-278) in a sample. Aspects of the subject disclosure include methods of designing and/or producing any diagnostic nucleic acid molecules exemplified or suggested herein.

This application also discloses plant breeding incorporating any of these methods. In some embodiments, the subject event can be "stacked" with other traits (such as other herbicide tolerance gene(s) and/or gene(s) that encode insect-inhibitory proteins, for example. Plant lines comprising the subject event can be detected using sequences disclosed and suggested herein.

In some embodiments, this application discloses the identification of herbicide-tolerant corn lines. The subject application discloses detecting the presence of the subject event in order to determine whether progeny of a sexual cross contain the event of interest. In addition, a method for detecting the event is disclosed and is helpful, for example, for complying with regulations requiring the pre-market approval and labeling of foods derived from recombinant crop plants, for example.

The subject invention relates in part to a fluorescence-based endpoint TaqMan PCR assay utilizing the endogenous invertase gene as a reference (copy number) control for high-throughput zygosity analysis of the AAD-1 maize event. The subject invention further relates, in part, to the discovery of a preferred reference gene, *invertase.* Several reference genes were identified as possible options.

The subject invention also relates in part to the development of a biplex endpoint TaqMan PCR for AAD-1 event specific zygosity analysis. Further, the subject invention relates in part to the development of AAD-1 breeding test kits.

Endpoint TaqMan assays are based on a plus/minus strategy, by which a "plus" signifies the sample is positive for the assayed gene and a "minus" signifies the sample is negative for the assayed gene. These assays typically utilize two sets of oligonucleotides for identifying the AAD-1 transgene sequence and the wild-type gene sequence respectively, as well as dual-labeled probes to measure the content of transgene and wild type sequence.

Although the Invader assay has been a robust technique for characterizing events, it is very sensitive to DNA quality. In addition, the assay requires a high quantity of DNA. Invader also requires an additional denaturing step which, if not handled properly, can render the Invader assay unsuccessful. Additionally, the longer assay time of the Invader assay is limited in its flexibility to efficiently handle large numbers of AAD-1 samples for analysis in a commercial setting. One main advantage of the subject invention is time savings and elimination of the denaturing step.

The subject Endpoint TaqMan analysis for detecting AAD-1 events offers surprising advantages over Invader, particularly in analyzing large number of samples.

This invention can impact the development and characterization of AAD-1 herbicide tolerance traits in crops including corn, soybean, and cotton.

Definitions and examples are provided herein to help describe the present invention and to guide those of ordinary skill in the art to practice the invention. Unless otherwise noted, terms are to be understood according to conventional usage by those of ordinary skill in the relevant art. The nomenclature for DNA bases as set forth at 37 CFR §1.822 is used. As used herein, the term "progeny" denotes the offspring of any generation of a parent plat which comprises AAD-1 corn event DAS-40278-9.

A transgenic "event" is produced by transformation of plant cells with heterologous DNA, *i.e*., a nucleic acid construct that includes a transgene of interest, regeneration of a population of plants resulting from the insertion of the transgene into the genome of the plant, and selection of a particular plant characterized by insertion into a particular genome location. The term "event" refers to the original transformant and progeny of the transformant that include the heterologous DNA. The term "event" also refers to progeny produced by a sexual outcross between the transformant and another variety that includes the genomic/transgene DNA. Even after repeated back-crossing to a recurrent parent, the inserted transgene DNA and flanking genomic DNA (genomic/transgene DNA) from the transformed parent is present in the progeny of the cross at the same chromosomal location. The term "event" also refers to DNA from the original transformant and progeny thereof comprising original transformant and progeny resulting from selfing) and a parental line that does not contain the inserted DNA.

A "junction sequence" spans the point at which DNA inserted into the genome is linked to DNA from the corn native genome flanking the insertion point. Included are the DNA sequences that span the insertions in herein-described corn events and similar lengths of flanking DNA.

The subject application discloses the identification of the subject event. Related PCR primers and amplicons are included in the invention. These molecules can be used to detect or identify commercialized transgenic corn varieties or lines derived from the subject transgenic corn lines.

The entire sequence of the insert, together with portions of the respective flanking sequences, are provided herein as SEQ ID NO: 1. The coordinates of the insert and flanking sequences for this event with respect to SEQ ID NO:1 (8557 basepairs total) are printed below.

| | **5' Flanking** | **Insert** | **3'Flanking** |
|---|---|---|---|
| residue #s (SEQ:29): | 1-1873 | 1874-6689 | 6690-8557 |
| length (bp): | 1873 bp | 4816 bp | 1868 bp |
| | | | |

The components of the AAD-1 insert and flanking sequences for this event are further illustrated in **Figures 1 through 3****.**

Detection techniques of the subject invention can be used in conjunction with plant breeding, to determine which progeny plants comprise a given event, after a parent plant comprising an event of interest is crossed with another plant line in an effort to impart one or more additional traits of interest in the progeny. The subject methods are useful in, for example, corn breeding programs as well as quality control, especially for commercialized transgenic cornseeds. This can also benefit product registration and product stewardship. These methods can be used for accelerated breeding strategies.

In some embodiments, the fluorescence-based end-point TaqMan assay for zygosity analysis allows the results to be directly read in a plate reader for identification of the AAD-1 event in corn and the reference gene.

The subject application discloses breeding applications such as testing the introgression of the AAD-1 event into other corn lines.

Detection methods and kits of the subject application can be used to identify events according to the subject application. Methods and kits of the subject invention can be used for accelerated breeding strategies and to establish linkage data.

Detection techniques of the subject application are especially useful in conjunction with plant breeding, to determine which progeny plants comprise a given event, after a parent plant comprising an event of interest is crossed with another plant line in an effort to impart one or more additional traits of interest in the progeny. These Taqman PCR analysis methods benefit maize breeding programs as well as quality control, especially for commercialized transgenic maize seeds. Taqman PCR detection kits for these transgenic maize lines can also now be made and used. This can also benefit product registration and product stewardship.

Still further, subject methods can be used to study and characterize transgene integration processes, genomic integration site characteristics, event sorting, stability of transgenes and their flanking sequences, and gene expression (especially related to gene silencing, transgene methylation patterns, position effects, and potential expression-related elements such as MARS [matrix attachment regions], and the like).

As used herein, the term "corn" means maize (*Zea mays*) and includes all varieties thereof that can be bred with corn.

This application further discloses processes of making crosses and using methods of the subject invention. For example, the subject application discloses a method for producing an F₁ hybrid seed by crossing an exemplified plant with a different (*e*.*g*. in-bred parent) plant, harvesting the resultant hybrid seed, and detecting for the subject event. Characteristics of the resulting plants may also be improved by incorporating methods of the subject invention.

A herbicide-tolerant corn plant can be bred by first sexually crossing a first parental corn plant consisting of a corn plant grown from seed of a line referred to herein, and a second parental corn plant, thereby producing a plurality of first progeny plants; and then selecting a first progeny plant that is resistant to a herbicide (or that possesses a subject event); and selfing the first progeny plant, thereby producing a plurality of second progeny plants; and then selecting from the second progeny plants a plant that is resistant to a herbicide (or that possesses at least one of the events). These steps can further include the back-crossing of the first progeny plant or the second progeny plant to the second parental corn plant or a third parental corn plant. A corn crop comprising corn seeds of the subject invention, or progeny thereof, can then be planted.

It is also to be understood that two different transgenic plants can also be mated to produce offspring that contain two independently segregating added, exogenous genes. Selfing of appropriate progeny can produce plants that are homozygous for both added, exogenous genes. Back-crossing to a parental plant and out-crossing with a non-transgenic plant are also contemplated, as is vegetative propagation. Other breeding methods commonly used for different traits and crops are known in the art. Backcross breeding has been used to transfer genes for a simply inherited, highly heritable trait into a desirable homozygous cultivar or inbred line, which is the recurrent parent. The source of the trait to be transferred is called the donor parent. The resulting plant is expected to have the attributes of the recurrent parent (*e*.*g*., cultivar) and the desirable trait transferred from the donor parent. After the initial cross, individuals possessing the phenotype of the donor parent are selected and repeatedly crossed (backcrossed) to the recurrent parent. The resulting parent is expected to have the attributes of the recurrent parent (*e*.*g*., cultivar) and the desirable trait transferred from the donor parent.

The present invention can be used in conjunction with a marker assisted breeding (MAB) method. Likewise, DNA molecules of the present invention can be used with other methods (such as, AFLP markers, RFLP markers, RAPD markers, SNPs, and SSRs) that identify genetically linked agronomically useful traits. The herbicide-resistance trait can be tracked in the progeny of a cross (or progeny thereof and any other corn cultivar or variety) using the MAB methods. The methods of the present invention can be used to identify any corn variety having the subject event.

Methods disclosed in the subject application include a method of producing a herbicide-tolerant corn plant wherein said method comprises breeding with a plant having a subject event. Preferred methods further comprise selecting progeny of said cross by analyzing said progeny for an event detectable according to the subject invention. For example, the subject invention can be used to track the subject event through breeding cycles with plants comprising other desirable traits, such as agronomic traits. Plants comprising the subject event and the desired trait can be detected, identified, selected, and quickly used in further rounds of breeding, for example. The subject event / trait can also be combined through breeding, and tracked according to the subject invention, with an insect resistant trait(s) and/or with further herbicide tolerance traits. One preferred embodiment of the latter is a plant comprising the subject event combined with a gene encoding resistance to an imidazolinone herbicide, glyphosate, and/or glufosinate. A dicamba tolerance gene can be used in some embodiments.

Thus, the subject invention can be combined with, for example, traits encoding glyphosate resistance (*e.g.,* resistant plant or bacterial *EPSPS, GOX, GAT*), glufosinate resistance (*e.g., Pat, bar*), acetolactate synthase (ALS)-inhibiting herbicide resistance (*e.g.,* imidazolinones [such as imazethapyr], sulfonylureas, triazolopyrimidine sulfonanilide, pyrmidinylthiobenzoates, and other chemistries [*Csr1*, *SurA, et al.*]), bromoxynil resistance (*e.g., Bxn*), resistance to inhibitors ofHPPD (4-hydroxlphenyl-pyruvate-dioxygenase) enzyme, resistance to inhibitors of phytoene desaturase (PDS), resistance to photosystem II inhibiting herbicides (*e.g., psbA),* resistance to photosystem I inhibiting herbicides, resistance to protoporphyrinogen oxidase IX (PPO)-inhibiting herbicides (*e.g*., *PPO-1),* resistance to phenylurea herbicides (*e.g., CYP76B1*), dicamba-degrading enzymes (*see, e.g.,* US 20030135879), and others could be stacked alone or in multiple combinations to provide the ability to effectively control or prevent weed shifts and/or resistance to any herbicide of the aforementioned classes.

Regarding additional herbicides, some additional preferred ALS (also known as AHAS) inhibitors include the triazolopyrimidine sulfonanilides (such as cloransulam-methyl, diclosulam, florasulam, flumetsulam, metosulam, and penoxsulam), pyrimidinylthiobenzoates (such as bispyribac and pyrithiobac), and flucarbazone. Some preferred HPPD inhibitors include mesotrione, isoxaflutole, and sulcotrione. Some preferred PPO inhibitors include flumiclorac, flumioxazin, flufenpyr, pyraflufen, fluthiacet, butafenacil, carfentrazone, sulfentrazone, and the diphenylethers (such as acifluorfen, fomesafen, lactofen, and oxyfluorfen).

As used herein, a "line" is a group of plants that display little or no genetic variation between individuals for at least one trait. Such lines may be created by several generations of self-pollination and selection, or vegetative propagation from a single parent using tissue or cell culture techniques.

As used herein, the terms "cultivar" and "variety" are synonymous and refer to a line which is used for commercial production.

"Stability" or "stable" means that with respect to the given component, the component is maintained from generation to generation and, preferably, at least three generations at substantially the same level, *e*.*g*., preferably ±15%, more preferably ±10%, most preferably ±5%. The stability may be affected by temperature, location, stress and the time of planting. Comparison of subsequent generations under field conditions should produce the component in a similar manner.

"Commercial Utility" is defined as having good plant vigor and high fertility, such that the crop can be produced by farmers using conventional farming equipment, and the oil with the described components can be extracted from the seed using conventional crushing and extraction equipment. To be commercially useful, the yield, as measured by seed weight, oil content, and total oil produced per acre, is within 15% of the average yield of an otherwise comparable commercial maize variety without the premium value traits grown in the same region.

"Agronomically elite" means that a line has desirable agronomic characteristics such as yield, maturity, disease resistance, and the like, in addition to insect resistance due to the subject event(s). Agronomic traits, taken individually or in any combination.

As one skilled in the art will recognize in light of this disclosure, preferred embodiments of detection kits, for example, can include probes and/or primers. For example, this includes a polynucleotide probes, primers, and/or amplicons as indicated herein.

One skilled in the art will also recognize that primers and probes can be designed to hybridize, under a range of standard hybridization and/or PCR conditions, to a segment of SEQ ID NO: 1 (or the complement), and complements thereof, wherein the primer or probe is not perfectly complementary to the exemplified sequence. That is, some degree of mismatch can be tolerated. For an approximately 20 nucleotide primer, for example, typically one or two or so nucleotides do not need to bind with the opposite strand if the mismatched base is internal or on the end of the primer that is opposite the amplicon. Various appropriate hybridization conditions are provided below. Synthetic nucleotide analogs, such as inosine, can also be used in probes. Peptide nucleic acid (PNA) probes, as well as DNA and RNA probes, can also be used. What is important is that such probes and primers are diagnostic for (able to uniquely identify and distinguish) the presence of an event of the subject invention.

The components of each of the "inserts" are illustrated in **Figures 1 through 3****.** The DNA polynucleotide sequences of these components, or fragments thereof, can be used as DNA primers or probes in the methods of the present invention.

DNA sequences that comprise a contiguous fragment of the novel transgene/genomic insertion region are disclosed herein. Included are DNA sequences that comprise a sufficient length of polynucleotides of transgene insert sequence and a sufficient length of polynucleotides of corn genomic sequence and/or sequences that are useful as primer sequences for the production of an amplicon product diagnostic for one or more of these corn plants.

Related embodiments pertain to DNA sequences that comprise at least 2, 3, 4, 5, 6, 7, 8, 9, 10, 11, 12, 13, 14, 15, 16, 17, 18, 19, 20, 21, 22, 23, 24, 25, or more contiguous nucleotides of a transgene portion of a DNA sequence identified herein, or complements thereof. Such sequences can be useful as DNA primers in DNA amplification methods. The amplicons produced using these primers are diagnostic for the corn event referred to herein. Therefore, the invention also includes amplicons and amplicons produced by such DNA primers and homologous primers.

This application discloses methods of detecting the presence of DNA, in a sample, that corresponds to the corn event referred to herein. Such methods can comprise: (a) contacting the sample comprising DNA with a primer set that, when used in a nucleic acid amplification reaction with DNA from at least one of these corn events, produces an amplicon that is diagnostic for said event(s); (b) performing a nucleic acid amplification reaction, thereby producing the amplicon; and (c) detecting the amplicon.

Further detection methods of the subject application discloses a method of detecting the presence of a DNA, in a sample, corresponding to at least one of said events, wherein said method comprises: (a) contacting the sample comprising DNA with a probe that hybridizes under stringent hybridization conditions with DNA from at least one of said corn events and which does not hybridize under the stringent hybridization conditions with a control corn plant (non-event-of-interest DNA); (b) subjecting the sample and probe to stringent hybridization conditions; and (c) detecting hybridization of the probe to the DNA.

This application discloses methods of detecting the presence of DNA, in a sample, from at least one of the maize plants referred to herein. Such methods can comprise: (a) contacting the sample comprising DNA with a primer set that, when used in a nucleic acid amplification reaction, of the subject invention, with DNA from at least one of these maize events; (b) performing a TAQMAN PCR amplification reaction using a reference gene identified herein; and (c) analyzing the results.

In still further embodiments, the subject application discloses methods of producing a corn plant comprising the AAD-1 event of the subject invention, wherein said method comprises the steps of: (a) sexually crossing a first parental corn line (comprising an expression cassettes of the present invention, which confers said herbicideresistance trait to plants of said line) and a second parental corn line (that lacks this herbicide tolerance trait) thereby producing a plurality of progeny plants; and (b) selecting a progeny plant by the use of molecular markers. Such methods may optionally comprise the further step of back-crossing the progeny plant to the second parental corn line to producing a true-breeding corn plant that comprises said herbicide tolerance trait.

According to another aspect of the invention, methods of determining the zygosity of progeny of a cross involving the subject event are provided. Said methods can comprise contacting a sample, comprising corn DNA, with a primer set of the subject invention. Said primers, when used in a nucleic-acid amplification reaction with genomic DNA from at least one of said corn events, produce a first amplicon that is diagnostic for at least one of said corn events. Such methods further comprise performing a nucleic acid amplification reaction, thereby producing the first amplicon detecting the first amplicon; and contacting the sample comprising corn DNA with said primer set, when used in a nucleic-acid amplification reaction with genomic DNA from corn plants, produces a second amplicon comprising the native corn genomic DNA homologous to the corn genomic region; and performing a nucleic acid amplification reaction, thereby producing the second amplicon. The methods further comprise detecting the second amplicon, and comparing the first and second amplicons in a sample, wherein the presence of both amplicons indicates that the sample is heterozygous for the transgene insertion.

DNA detection kits can be developed using the compositions disclosed herein and methods well known in the art of DNA detection. The kits are useful for identification of the subject corn event DNA in a sample and can be applied to methods for breeding corn plants containing this DNA. The kits contain DNA sequences homologous or complementary to the amplicons, for example, disclosed herein, or to DNA sequences homologous or complementary to DNA contained in the transgene genetic elements of the subject events. These DNA sequences can be used in DNA amplification reactions or as probes in a DNA hybridization method. The kits may also contain the reagents and materials necessary for the performance of the detection method.

A "probe" is an isolated nucleic acid molecule which is attached to a conventional detectable label or reporter molecule (such as a radioactive isotope, ligand, chemiluminescent agent, or enzyme). Such a probe is complementary to a strand of a target nucleic acid, in the case of the present invention, to a strand of genomic DNA from one of said corn events, whether from a corn plant or from a sample that includes DNA from the event. Probes according to the present invention include not only deoxyribonucleic or ribonucleic acids but also polyamides and other probe materials that bind specifically to a target DNA sequence and can be used to detect the presence of that target DNA sequence.

"Primers" are isolated/synthesized nucleic acids that are annealed to a complementary target DNA strand by nucleic acid hybridization to form a hybrid between the primer and the target DNA strand, then extended along the target DNA strand by a polymerase, *e*.*g*., a DNA polymerase. Primer pairs of the present invention refer to their use for amplification of a target nucleic acid sequence, *e.g.,* by the polymerase chain reaction (PCR) or other conventional nucleic-acid amplification methods.

Probes and primers are generally 5, 6, 7, 8, 9, 10, 11, 12, 13, 14, 15, 16, 17, 18, 19,20,21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, or 500 polynucleotides or more in length. Such probes and primers hybridize specifically to a target sequence under high stringency hybridization conditions. Preferably, probes and primers according to the present invention have complete sequence similarity with the target sequence, although probes differing from the target sequence and that retain the ability to hybridize to target sequences may be designed by conventional methods.

Methods for preparing and using probes and primers are described, for example, in Molecular Cloning: A Laboratory Manual, 2nd ed., vol. 1-3, ed. Sambrook et al., Cold Spring Harbor Laboratory Press, Cold Spring Harbor, N.Y., 1989. PCR-primer pairs can be derived from a known sequence, for example, by using computer programs intended for that purpose.

Primers and probes based on the flanking DNA and insert sequences disclosed herein can be used to confirm (and, if necessary, to correct) the disclosed sequences by conventional methods, e.g., by re-cloning and sequencing such sequences.

The nucleic acid probes and primers of the present invention hybridize under stringent conditions to a target DNA sequence. Any conventional nucleic acid hybridization or amplification method can be used to identify the presence of DNA from a transgenic event in a sample. Nucleic acid molecules or fragments thereof are capable of specifically hybridizing to other nucleic acid molecules under certain circumstances. As used herein, two nucleic acid molecules are said to be capable of specifically hybridizing to one another if the two molecules are capable of forming an anti-parallel, double-stranded nucleic acid structure. A nucleic acid molecule is said to be the "complement" of another nucleic acid molecule if they exhibit complete complementarity. As used herein, molecules are said to exhibit "complete complementarity" when every nucleotide of one of the molecules is complementary to a nucleotide of the other. Two molecules are said to be "minimally complementary" if they can hybridize to one another with sufficient stability to permit them to remain annealed to one another under at least conventional "low-stringency" conditions. Similarly, the molecules are said to be "complementary" if they can hybridize to one another with sufficient stability to permit them to remain annealed to one another under conventional "high-stringency" conditions. Conventional stringency conditions are described by Sambrook *et al.,* 1989. Departures from complete complementarity are therefore permissible, as long as such departures do not completely preclude the capacity of the molecules to form a double-stranded structure. In order for a nucleic acid molecule to serve as a primer or probe it need only be sufficiently complementary in sequence to be able to form a stable double-stranded structure under the particular solvent and salt concentrations employed.

As used herein, a substantially homologous sequence is a nucleic acid sequence that will specifically hybridize to the complement of the nucleic acid sequence to which it is being compared under high stringency conditions. The term "stringent conditions" is functionally defined with regard to the hybridization of a nucleic-acid probe to a target nucleic acid (*i.e.,* to a particular nucleic-acid sequence of interest) by the specific hybridization procedure discussed in Sambrook *et al.,* 1989, at 9.52-9.55. *See also,* Sambrook *et al*., 1989 at 9.47-9.52 and 9.56-9.58. Accordingly, the nucleotide sequences of the invention may be used for their ability to selectively form duplex molecules with complementary stretches of DNA fragments.

Depending on the application envisioned, one can use varying conditions of hybridization to achieve varying degrees of selectivity of probe towards target sequence. For applications requiring high selectivity, one will typically employ relatively stringent conditions to form the hybrids, *e*.*g*., one will select relatively low salt and/or high temperature conditions, such as provided by about 0.02 M to about 0.15 M NaCl at temperatures of about 50° C to about 70° C. Stringent conditions, for example, could involve washing the hybridization filter at least twice with high-stringency wash buffer (0.2X SSC, 0.1% SDS, 65° C). Appropriate stringency conditions which promote DNA hybridization, for example, 6.0X sodium chloride/sodium citrate (SSC) at about 45° C, followed by a wash of 2.0X SSC at 50° C are known to those skilled in the art. For example, the salt concentration in the wash step can be selected from a low stringency of about 2.0X SSC at 50° C to a high stringency of about 0.2X SSC at 50° C. In addition, the temperature in the wash step can be increased from low stringency conditions at room temperature, about 22° C, to high stringency conditions at about 65° C. Both temperature and salt may be varied, or either the temperature or the salt concentration may be held constant while the other variable is changed. Such selective conditions tolerate little, if any, mismatch between the probe and the template or target strand. Detection of DNA sequences via hybridization is well-known to those of skill in the art, and the teachings of U.S. Patent Nos. 4,965,188 and 5,176,995 are exemplary of the methods of hybridization analyses.

In a particularly preferred embodiment, a nucleic acid of the present invention will specifically hybridize to one or more of the primers (or amplicons or other sequences) exemplified or suggested herein, including complements and fragments thereof, under high stringency conditions. In one aspect of the present invention, a nucleic acid molecule of the present invention has the nucleic acid sequence set forth in SEQ ID NOS:2-7, or complements and/or fragments thereof.

In another aspect of the present invention, a marker nucleic acid molecule of the present invention shares between 80% and 100% or 90% and 100% sequence identity with such nucleic acid sequences. In a further aspect of the present invention, a marker nucleic acid molecule of the present invention shares between 95% and 100% sequence identity with such sequence. Such sequences may be used as markers in plant breeding methods to identify the progeny of genetic crosses. The hybridization of the probe to the target DNA molecule can be detected by any number of methods known to those skilled in the art, these can include, but are not limited to, fluorescent tags, radioactive tags, antibody based tags, and chemiluminescent tags.

Regarding the amplification of a target nucleic acid sequence (*e.g*., by PCR) using a particular amplification primer pair, "stringent conditions" are conditions that permit the primer pair to hybridize only to the target nucleic-acid sequence to which a primer having the corresponding wild-type sequence (or its complement) would bind and preferably to produce a unique amplification product, the amplicon.

The term "specific for (a target sequence)" indicates that a probe or primer hybridizes under stringent hybridization conditions only to the target sequence in a sample comprising the target sequence.

As used herein, "amplified DNA" or "amplicon" refers to the product of nucleic-acid amplification of a target nucleic acid sequence that is part of a nucleic acid template. For example, to determine whether the corn plant resulting from a sexual cross contains transgenic event genomic DNA from the corn plant of the present invention, DNA extracted from a corn plant tissue sample may be subjected to nucleic acid amplification method using a primer pair that includes a primer derived from flanking sequence in the genome of the plant adjacent to the insertion site of inserted heterologous DNA, and a second primer derived from the inserted heterologous DNA to produce an amplicon that is diagnostic for the presence of the event DNA. The amplicon is of a length and has a sequence that is also diagnostic for the event. The amplicon may range in length from the combined length of the primer pairs plus one nucleotide base pair, and/or the combined length of the primer pairs plus about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139,140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, or 500, 750,1000,1250, 1500, 1750, 2000, or more nucleotide base pairs (plus or minus any of the increments listed above). Alternatively, a primer pair can be derived from flanking sequence on both sides of the inserted DNA so as to produce an amplicon that includes the entire insert nucleotide sequence. A member of a primer pair derived from the plant genomic sequence may be located a distance from the inserted DNA sequence. This distance can range from one nucleotide base pair up to about twenty thousand nucleotide base pairs. The use of the term "amplicon" specifically excludes primer dimers that may be formed in the DNA thermal amplification reaction.

Nucleic-acid amplification can be accomplished by any of the various nucleic-acid amplification methods known in the art, including the polymerase chain reaction (PCR). A variety of amplification methods are known in the art and are described, *inter alia*, in U.S. Patent No. 4,683,195 and U.S. Patent No. 4,683,202. PCR amplification methods have been developed to amplify up to 22 kb of genomic DNA. These methods as well as other methods known in the art of DNA amplification may be used in the practice of the present invention. The sequence of the heterologous transgene DNA insert or flanking genomic sequence from a subject corn event can be verified (and corrected if necessary) by amplifying such sequences from the event using primers derived from the sequences provided herein followed by standard DNA sequencing of the PCR amplicon or of the cloned DNA.

The amplicon produced by these methods may be detected by a plurality of techniques. Agarose gel electrophoresis and staining with ethidium bromide is a common well known method of detecting DNA amplicons. Another such method is Genetic Bit Analysis where an DNA oligonucleotide is designed which overlaps both the adjacent flanking genomic DNA sequence and the inserted DNA sequence. The oligonucleotide is immobilized in wells of a microwell plate. Following PCR of the region of interest (using one primer in the inserted sequence and one in the adj acent flanking genomic sequence), a single-stranded PCR product can be hybridized to the immobilized oligonucleotide and serve as a template for a single base extension reaction using a DNA polymerase and labeled ddNTPs specific for the expected next base. Readout may be fluorescent or ELISA-based. A signal indicates presence of the insert/flanking sequence due to successful amplification, hybridization, and single base extension.

The following abbreviations are used unless otherwise indicated.
- AAD-1: aryloxyalkanoate dioxygenase-1
- bp: base pair
- °C: degrees Celcius
- DNA: deoxyribonucleic acid
- DIG: digoxigenin
- EDTA: ethylenediaminetetraacetic acid
- kb: kilobase
- µg: microgram
- µL: microliter
- mL: milliliter
- M: molar mass
- OLP: overlapping probe
- PCR: polymerase chain reaction
- PTU: plant transcription unit
- SDS: sodium dodecyl sulfate
- SOP: standard operating procedure
- SSC: a buffer solution containing a mixture of sodium chloride and sodium citrate, pH 7.0
- TBE: a buffer solution containing a mixture of Tris base, boric acid and EDTA, pH 8.3
- V: volts

### EXAMPLES

### Example 1. Event Specific Taqman Assay

An event specific Taqman assay was developed to detect the presence of maize event DAS-40278-9 and to determine zygosity status of plants in breeding populations. To develop an event specific assay, specific Taqman primers and probes were designed according to the DNA sequences located in the 5' insert-to-plant junction. For specific detection of DAS-40278-9, a 73-bp DNA fragment that spans this 5'-integration junction was amplified using two specific primers. The amplification of this PCR product was measured by a target-specific MGB probe synthesized by Applied Biosystems containing the FAM reporter at its 5'end. Specificity of this Taqman detection method for AAD-1 corn event DAS-40278-9 was tested against 16 different AAD-1 corn events and non-transgenic corn variety in duplex format with the corn specific endogenous reference gene, Invertase.

### Example 1.1. gDNA Isolation

gDNA samples of 16 different AAD-1 corn events and non-transgenic corn varieties were tested in this study. gDNA was extracted with two approaches, Qiagen kit or CTAB. For the gDNA samples extracted with the Qiagen kit, eight corn fresh leaf discs were used for gDNA extraction according to a modified Qiagen DNeasy 96 Plant Kit protocol. For the gDNA samples extracted by using CTAB procedure, about 0.3 g lyophilized leaf tissue was used following a protocol from Permingeat et al., 1998. gDNA was quantified with the Pico Green method according to vendor's instructions (Molecular Probes, Eugene, OR). The gDNA samples were diluted with DNase-free water resulting in a concentration of 10 ng/µL for the purpose of this study.

### Example 1.2. Taqman Assay and Results

Specific Taqman primers and probes were designed for the DAS-40278-9 event specific Taqman assay. These reagents can be used with the conditions listed below to detect AAD-1 corn event DAS-40278-9. Table 1 lists the primer and probe sequences that were developed specifically for the detection of event DAS-40278-9.

**Table 1. PCR Primers and Probes**

| **Event Target Reaction** | | |
|---|---|---|
| | | |
| **Name** | **Description** | **5' to 3' sequence** |
| Corn278-F | Forward Primer | Seq ID NO:2: 5' - ATTCTGGCTTTGCTGTAAATCGT - 3' |
| Corn278-R | Reverse Primer | Seq ID NO: 3:5' - TTACAATCAACAGCACCGTACCTT - 3' |
| Corn278-Probe | Probe | Seq ID NO: 4: 5' - FAM- CTAACCTTCATTGTATTCC-MGB -3' |

| **Invertase Reference System Reaction** | | |
|---|---|---|
| **Name** | **Description** | **5' to 3' sequence** |
| IVF | Forward Primer | Seq ID NO: 5: 5' - TGGCGGACGACGACTTGT - 3' |
| IVR | Reverse Primer | Seq ID NO: 6: 5' - AAAGTTTGGAGGCTGCCGT - 3' |
| IV-Probe | Probe | Seq ID NO: 7: 5'-HEX-CGAGCAGACCGCCGTGTACTTCTACC-BHQ2 - 3' |

The multiplex PCR conditions for amplification are as follows: 1X PCR buffer, .5 - 2.5 mM MgCl₂, .2 mM dNTP, 0.2 µM Primer Corn-278-F, 0.2 µM Primer Corn-278-R, 0.2 µM Primer_IV-F, 0.2 µM Primer_IV-R, 0.08 µM Probe_Corn-278-Probe, 0.08 uM Probe_IV-probe, 40 U/mL HotStart Taq, 0.6 to 2.4 ug/mL DNA in a total reaction of 25 µl. Various concentrations of MgCl₂ and DNA were tested. Concentrations of .5 mM, 1.0 mM, 1.8 mM, and 2.5 mM of MgCl₂ were used. The cocktail was amplified using the following conditions: i) 95°C for 15 min., ii) 95°C for 20 sec, iii) 60°C for 60 sec, iv) repeat step ii-iii for 50 cycles, v) 4°C hold. The Real time PCR was carried out on Bio-rad iCycler™ system. Data analysis was based on measurement of the cycle threshold (CT), which is the PCR cycle number when the fluorescence measurement reaches a set value. CT value was calculated automatically by iCycler software.

The amplicon sequences generated using the above primers were as follows:
278F and 278R:
   ttacaatcaacagcaccgtaccttgaagcggaatacaatgaaggttagctacgatttacagcaaagccagaat
      (SEQ ID NO:8)
IVF and IVR:
   tggcggacgacgacttgtccgagcagaccgccgtgtacttctacctgctcaagggcacggacggcagcctccaaacttt
      (SEQ ID NO:9)

The Taqman detection method for AAD-1 corn event DAS-40278-9 was tested against 16 different AAD-1 corn events and non-transgenic corn variety in duplex format with corn specific endogenous Invertase as a reference gene. This assay specifically detected the AAD-1 corn event DAS-40278-9 and did not produce or amplify any false-positive results from the controls (i.e. the 16 different AAD-1 corn events and non-transgenic corn varieties). The event specific primers and probes can be used for the detection of the AAD-1 corn event DAS-40278-9 and these conditions and reagents are applicable for zygosity assays.

## Claims

1. A method for determining zygosity of a corn plant comprising an AAD-1 corn event DAS-40278-9, said event comprising a transgene construct comprising an AAD-1 gene, said transgene construct being flanked by a 5' flanking corn genomic DNA and a 3' flanking corn genomic DNA, said method comprising:
obtaining a DNA sample of genomic DNA from said corn plant;
producing a contacted sample by contacting said DNA sample with
a. a first event primer and a second event primer, wherein said first event primer specifically binds said transgene construct as defined by residues 1874-6689 of SEQ ID NO:29, said second event primer specifically binds said 5' corn genomic flanking DNA as defined by residues 1-1873 of SEQ ID NO:29 or said 3' corn genomic flanking DNA as defined by residues 6690-8557 of SEQ ID NO:29, and wherein said first event primer and said second event primer produce an event amplicon when subjected to a PCR reaction
b. a reference forward primer and a reference reverse primer that produce a reference amplicon from an endogenous corn reference gene when subjected to a PCR reaction, wherein said reference gene:
i. is an endogenous Zea mays invertase gene; or
ii. comprises a sequence selected from the group consisting of SEQ ID NO:5, SEQ ID NO:6, and SEQ ID NO:7 or a sequence having at least 90 % sequence identity to any one of the aforementioned sequences,
c. a fluorescent event probe that hybridizes with said event amplicon
d. a fluorescent reference probe that hybridizes with said reference amplicon;
subjecting said contacted sample to a fluorescence-based hydrolysis probe assay which
releases the fluorescent moiety of the probe from the quenching moiety of the probe;
quantitating said florescent event probe that hybridized to said event amplicon;
quantitating said florescent reference probe that hybridized to said reference amplicon; comparing amounts of hybridized florescent event probe to hybridized florescent reference probe; and
determining zygosity of DAS-40278-9 by comparing florescence ratios of hybridized fluorescent event probe and hybridized fluorescent reference probe.

2. The method of claim 1 wherein said amplicons consist of 50-100 residues.

3. The method of claim 1 wherein said second event primer binds residues 1673-1873 of SEQ ID NO:29 or the complement thereof or 6690-6890 of SEQ ID NO:29.

4. The method of claim 1 wherein said method is used for the identification of herbicide-resistant corn lines.

5. The method of claim 1 wherein said event amplicon is 73 basepairs.

6. The method of claim 1 wherein said reference primers comprise SEQ ID NO: 5 and SEQ ID NO:6, and said reference probe comprises SEQ ID NO:7.

7. The method of claim 1 wherein said probes are labeled with a fluorescent dye and quencher.

8. The method of claim 7 wherein said event probe comprises FAM as said fluorescent dye at the 5' end of said event probe and an MGB quencher on the 3' end of said event probe.

9. The method of claim 1 wherein said reference amplicon is a 79 basepair fragment amplified by said primers.

10. The method of claim 1 wherein said event amplicon consist of SEQ ID NO:8 and said reference amplicon consists of SEQ ID NO:9.

11. The method of claim 1 wherein said event probe comprises SEQ ID NO:4.

12. The method of claim 1 wherein said event primers are selected from the group consisting of SEQ ID NO:2 and SEQ ID NO:3.

13. The method of claim 1 wherein results of said method are read directly in a plate reader.

14. The method of 1 wherein said DNA sample is obtained from a corn plant in a field.

15. A kit for performing the method of claim 1, said kit comprising a first event primer defined by SEQ ID NO: 2, a second event primer defined by SEQ ID NO: 3, a reference forward primer defined by SEQ ID NO: 5, a reference reverse primer defined by SEQ ID NO: 6, an event probe defined by SEQ ID NO: 4, and a reference probe defined by SEQ ID NO: 7.

## Patentansprüche

1. Ein Verfahren zur Bestimmung der Zygotizität einer Maispflanze umfassend ein AAD-1 Maisereignis DAS-40278-9, wobei besagtes Ereignis ein transgenes Konstrukt umfasst, welches ein AAD-1-Gen umfasst, wobei besagtes transgenes Konstrukt von einer 5' flankierenden genomischen Mais-DNA und einer 3' flankierenden genomischen Mais-DNA flankiert ist, wobei das Verfahren umfasst:
Erhalten einer DNA-Probe aus genomischer DNA der besagten Maispflanze;
Herstellen einer kontaktierten Probe durch kontaktieren besagter DNA-Probe mit
a. einem ersten Ereignisprimer und einem zweiten Ereignisprimer, wobei besagter erster Ereignisprimer spezifisch an besagtes Transgen Konstrukt bindet, welches durch die Reste 1874-6689 von SEQ ID NO: 29 definiert wird, wobei besagter zweiter Ereignisprimer spezifisch an besagte 5' flankierende genomische Mais-DNA, welche durch die Reste 1-1879 von SEQ ID NO: 29 definiert wird, oder an besagten 3' flankierende genomische Mais-DNA, welche durch die Reste 6690-8557 von SEQ ID NO: 29 definiert wird, bindet, und wobei besagter erster Ereignisprimer und besagter zweiter Ereignisprimer ein Ereignisamplikon produzieren, wenn sie einer PCR-Reaktion unterzogen werden
b. ein Referenz-Vorwärtsprimer und Referenz-Rückwärtsprimer, welche ein Referenzamplikon von einem endogenen Mais-Referenzgen erzeugen, wenn sie einer PCR-Reaktion unterzogen werden, wobei besagtes Referenzgen:
i. ein endogenes *Zea Mays* Invertasegen ist; oder
ii. eine Sequenz umfasst, die aus der Gruppe bestehend aus SEQ ID NO: 5, SEQ ID NO: 6, und SEQ ID NO: 7 oder einer Sequenz, die mindestens 90% Sequenz Identität zu einer der zuvor genannten Sequenzen hat, ausgewählt ist,
c. eine Fluoreszenz-Ereignis-Sonde, die mit besagtem Ereignisamplikon hybridisiert
d. eine Fluoreszenz-Referenz-Sonde, die mit besagtem Referenzamplikon hybridisiert;
Unterziehen besagter kontaktierter Probe einem Fluoreszenz-basierten Hydrolyse-Sonden Versuch, welcher die fluoreszierende Einheit der Sonde von der quenchenden Einheit der Sonde freisetzt;
Quantifizieren besagter Fluoreszenz-Ereignis Sonde, welche an besagtes Ereignisamplikon hybridisiert hat;
Quantifizieren besagter Fluoreszenz-Referenz Sonde, welche an besagtes Referenzamplikon hybridisiert hat;
Vergleichen der Mengen der hybridisierten Fluoreszenz-Ereignis-Sonde zu mit hybridisierten Fluoreszenz-Referenz-Sonde, und
Bestimmen der Zygotisität von DAS-40278-9 durch Vergleichen der Fluoreszenz-Verhältnisse hybridisierten Fluoreszenz-Ereignis-Sonden mit hybridisierten Fluoreszenz-Referenz-Sonden.

2. Verfahren gemäß Anspruch 1, wobei besagtes Amplikon aus 50-100 Resten besteht.

3. Verfahren gemäß Anspruch 1, wobei besagter Referenzprimer an die Reste 1673-1873 von SEQ ID NO: 29, oder an das Komplement dieser, oder 6690-6890 von SEQ ID NO: 29 bindet.

4. Verfahren nach Anspruch 1, wobei besagtes Verfahren zur Identifizierung von herbizidresistenten Maislinien verwendet wird.

5. Verfahren nach Anspruch 1, wobei besagtes Ereignisamplikon 73 Basenpaare aufweist.

6. Verfahren nach Anspruch 1, wobei besagte Referenzprimer SEQ ID NO: 5 und SEQ ID NO: 6 umfassen, und besagte Referenzsonde SEQ ID NO: 7 umfasst.

7. Verfahren nach Anspruch 1, wobei besagte Sonden mit einem fluoreszierenden Farbstoff und Quencher markiert ist.

8. Verfahren nach Anspruch 7, wobei besagte Ereignissonde FAM als Fluorezenz Farbstoff am 5' Ende von besagter Ereignissonde und einen MGB-Quencher am 3' Ende von besagter Ereignissonde umfasst.

9. Verfahren nach Anspruch 1, wobei besagtes Referenzamplikon ein 79 Basenpaar Fragment ist, das durch besagte Primer amplifiziert wurde.

10. Verfahren nach Anspruch 1, wobei besagtes Ereignisamplikon aus SEQ ID NO: 8 besteht und besagtes Referenzamplikon aus SEQ ID NO: 9 besteht.

11. Verfahren nach Anspruch 1, wobei besagte Ereignissonde SEQ ID NO: 4 umfasst.

12. Verfahren nach Anspruch 1, wobei besagte Ereignisprimer aus der Gruppe bestehend aus SEQ ID NO: 2 und SEQ ID NO: 3 ausgewählt sind.

13. Verfahren nach Anspruch 1, wobei die Ergebnisse von besagtem Verfahren direkt in einem Platten-Lesegerät ausgelesen wird.

14. Verfahren nach Anspruch 1, wobei besagte DNA Probe von einer Maispflanze in einem Feld erhalten wird.

15. Kit zur Durchführung des Verfahrens nach Anspruch 1, wobei besagtes Kit einen ersten Ereignisprimer, der durch SEQ ID NO: 2 definiert ist, einen zweiten Ereignisprimer, der durch SEQ ID NO: 3 definiert ist, einen vorwärts Referenzprimer, der durch SEQ ID NO: 5 definiert ist, einen rückwärts Referenzprimer, der durch SEQ ID NO: 6 definiert ist, eine Ereignissonde, die durch SEQ ID NO: 4 definiert ist, und eine Referenzsonde, die durch SEQ ID NO: 7 definiert ist, umfasst.

## Revendications

1. Procédé de détermination de la zygosité d'un plant de maïs DAS-40278-9 porteur d'une séquence « Event » produisant de l'AAD-1, laquelle séquence comprend une construction transgénique comportant un gène d'AAD-1, laquelle construction transgénique est flanquée d'un ADN génomique de maïs de flanc 5' et d'un ADN génomique de maïs de flanc 3', et lequel procédé comporte les étapes suivantes :
- obtenir un échantillon d'ADN génomique dudit plant de maïs ;
- produire un échantillon post-contact, en mettant ledit échantillon d'ADN en contact avec
a) une première amorce pour séquence Event et une deuxième amorce pour séquence Event, laquelle première amorce pour séquence Event se lie spécifiquement à ladite construction transgénique, définie par les résidus 1874 à 6689 de la Séquence N° 29, laquelle deuxième amorce pour séquence Event se lie spécifiquement audit ADN génomique de maïs de flanc 5', défini par les résidus 1 à 1873 de la Séquence N° 29, ou audit ADN génomique de maïs de flanc 3', défini par les résidus 6690 à 8557 de la Séquence N° 29, et lesquelles première et deuxième amorces pour séquence Event produisent un amplicon de séquence Event quand on les fait réagir en PCR,
b) une amorce sens de référence et une amorce anti-sens de référence, qui produisent un amplicon de référence, à partir d'un gène endogène de maïs de référence, quand on les fait réagir en PCR, étant entendu que ledit gène de référence
i) est un gène endogène d'invertase de Zea mays,
ii) ou comporte une séquence choisie dans l'ensemble formé par les Séquences N° 5, N° 6 et N° 7, ou une séquence identique, à un degré d'au moins 90 %, à l'une des séquences mentionnées ci-dessus,
c) une sonde fluorescente pour séquence Event, qui s'hybride audit amplicon de séquence Event,
d) et une sonde fluorescente de référence, qui s'hybride audit amplicon de référence ;
- soumettre ledit échantillon post-contact à un dosage de sonde, reposant sur le phénomène de fluorescence, après hydrolyse où le fragment fluorescent de la sonde est séparé du fragment extincteur de la sonde ;
- quantifier ladite sonde fluorescente pour séquence Event qui s'est hybridée audit amplicon de séquence Event ;
- quantifier ladite sonde fluorescente de référence qui s'est hybridée audit amplicon de référence ;
- comparer les quantités de la sonde fluorescente pour séquence Event hybridée et de la sonde fluorescente de référence hybridée ;
- et déterminer la zygosité du plant DAS40278-9 en comparant les rapports de fluorescence de la sonde fluorescente pour séquence Event hybridée et de la sonde fluorescente de référence hybridée.

2. Procédé conforme à la revendication 1, dans lequel lesdits amplicons sont constitués de 50 à 100 résidus.

3. Procédé conforme à la revendication 1, dans lequel ladite deuxième amorce pour séquence Event se lie aux résidus 1673 à 1873 de la Séquence N° 29 ou à la séquence complémentaire de ce fragment, ou aux résidus 6690 à 6890 de la Séquence N° 29.

4. Procédé conforme à la revendication 1, lequel procédé est utilisé pour l'identification de lignées de maïs résistantes à un herbicide.

5. Procédé conforme à la revendication 1, dans lequel ledit amplicon de séquence Event est long de 73 paires de bases.

6. Procédé conforme à la revendication 1, dans lequel lesdites amorces de référence comportent la Séquence N° 5 et la Séquence N° 6, et ladite sonde de référence comporte la Séquence N° 7.

7. Procédé conforme à la revendication 1, dans lequel lesdites sondes sont marquées avec un colorant fluorescent et un extincteur de fluorescence.

8. Procédé conforme à la revendication 7, dans lequel ladite sonde pour séquence Event comprend une FAM, en tant que colorant fluorescent, à l'extrémité 5' de ladite sonde pour séquence Event, et un extincteur MGB à l'extrémité 3' de ladite sonde pour séquence Event.

9. Procédé conforme à la revendication 1, dans lequel ledit amplicon de référence est un fragment de 79 paires de bases amplifié par lesdites amorces.

10. Procédé conforme à la revendication 1, dans lequel ledit amplicon de séquence Event consiste en la Séquence N° 8 et ledit amplicon de référence consiste en la Séquence N° 9.

11. Procédé conforme à la revendication 1, dans lequel ladite sonde pour séquence Event consiste en la Séquence N° 4.

12. Procédé conforme à la revendication 1, dans lequel lesdites amorces pour séquence Event sont choisies dans l'ensemble constitué par la Séquence N° 2 et la Séquence N° 3.

13. Procédé conforme à la revendication 1, dont les résultats sont lus directement sur un lecteur de plaques.

14. Procédé conforme à la revendication 1, pour lequel ledit échantillon d'ADN est obtenu à partir d'un plant de maïs qui est dans un champ.

15. Trousse pour la mise en oeuvre d'un procédé conforme à la revendication 1, laquelle trousse comporte une première amorce pour séquence Event, définie par la Séquence N° 2, une deuxième amorce pour séquence Event, définie par la Séquence N° 3, une amorce sens de référence, définie par la Séquence N° 5, une amorce anti-sens de référence, définie par la Séquence N° 6, une sonde pour séquence Event, définie par la Séquence N° 4, et une sonde de référence, définie par la Séquence N° 7.


### SEQUENCE LISTING

<110> Dow AgroSciences LLC
<120> DETECTION OF AAD-1 EVENT DAS-40278-9
<130> DAS-P0169-01
<160> 9
<170> PatentIn version 3.5
<210> 1
   <211> 8557
   <212> DNA
   <213> Artificial Sequence
<220>
   <223> AAD-1 insert and flanking sequences
<400> 1
   actggtattt aatatacttt aataaatatt attagattcc tcgtcaaaga actttttaca      60
   atatatctat ttagaatcat atatgtcata gttttttttc taagagtcta gtttactagt      120
   aaaatccgac tcacattttt cgaacttggg atgcaacact taaatagtac aaaaccttgg     180
   tatgcagtat tttacattgt aagattcaaa atttctaaag cagtatatat atgtttccag     240
   aaacttatag atatagaaaa aacagagaga cgtatgcgaa aattcgataa aggtgtacat     300
   tggattcgca aggctaaata catatttatc gtggatccat gcagagtttg ggtaataaaa     360
   ttagatactt ccaatcatgt gccacataat cacgtaacat tagtaattta aatgacatta     420
   ccatgtccaa ctgatttaaa acacaaactc ttcttgaacc atatagtttg acaaaccaaa     480
   tatatataac tggagctact agttatgaat caattaaaaa ttactttgaa gattcaacgt     540
   agtgccagtt tggctctagc acatctaacc agaagggcta aggctggctt caacaggaac     600
   agccaaatcc gagatcgagc catttgccat ttttgggtag ttagtttaac tttcatatat     660
   cttcccatcc ttttttgcct agcctaaatg gctttgatgt tgaagaccat attaatttgc     720
   ttcagtggca ctaggacaac catattggct ttggctgacc cgttagagtt agcctaatgg     780
   gtggaagggg agggaagggg aggatcgatg gtggcatgag agaggggttg acgatcacga     840
   tgatgatgcg agtgaggagg agagggtggc gacgacacag gggagaaagg agagggacgc     900
   taggagcgtc aagggcgtgg gggaggggag ggtcggaggg atgaaggatg acctaaatat     960
   tattgttgag tgatagaggg ttattcaact atccgacccg tcgattttga tggtatgtta    1020
   aatttgtgtg tcatttgttt gatggattta gtaaaggtta tgggtctaga ggtgattttt    1080
   gttgggtggg ttttacagag tttaaactag cggattatat agtggtatag aagatatagt    1140
   tttattagaa catctccaaa atgtgactcg aaataatacc cccaaaattt aaaatactac    1200
   atcattttga taaaaaaggt aaagtagagc actgttggaa cagtttttaa aagttgtgcc    1260
   ctatatttta aaatagggta ctgatttaaa atattgttgt gggggataga tatccccggg     1320
   tccactagaa ggcgagaagg cctcgcgtgt ggccacgggc cagttacccc gcaaggccat 1380
   cccttcgtgg gtcgagctag aattactggt agaatgggct gaccgaagaa ggcaacagac 1440
   tcgagcccaa acaatccatc ggctcgtgcg ctatccacag aaactacccg actttccggc 1500
   gcatggcatc ctagaatatc ggggcgtatt agggatgagt cagcgagatt ttcggaagat 1560
   tagttcagtt tgttcgctat tatttaggag acatatgatc ctcatgtacg tatggagtgc 1620
   cccacggtcg tgtatataag gtccagaggg taccccatca tttctatcga ccatctacct 1680
   atctcatcag cttttctcca ttcaggagac ctcgcttgta acccaccaca tatagatcca 1740
   tcccaagaag tagtgtatta cgcctctcta agcggcccaa acttgcagaa aaccgcctat 1800
   ccctctctcg tgcgtccagc acgaaccatt gagttacaat caacagcacc gtaccttgaa 1860
   gcggaataca atgaaggtta gctacgattt acagcaaagc cagaatacaa tgaaccataa 1920
   agtgattgaa gctcgaaata tacgaaggaa caaatatttt taaaaaaata cgcaatgact 1980
   tggaacaaaa gaaagtgata tattttttgt tcttaaacaa gcatcccctc taaagaatgg 2040
   cagttttcct ttgcatgtaa ctattatgct cccttcgtta caaaaatttt ggactactat 2100
   tgggaacttc ttctgaaaat agtggccacc gcttaattaa ggcgcgccat gcccgggcaa 2160
   gcggccgctt aattaaattt aaatgtttaa actaggaaat ccaagcttgc atgcctgcag 2220
   atccccgggg atcctctaga gtcgacctgc agtgcagcgt gacccggtcg tgcccctctc 2280
   tagagataat gagcattgca tgtctaagtt ataaaaaatt accacatatt ttttttgtca 2340
   cacttgtttg aagtgcagtt tatctatctt tatacatata tttaaacttt actctacgaa 2400
   taatataatc tatagtacta caataatatc agtgttttag agaatcatat aaatgaacag 2460
   ttagacatgg tctaaaggac aattgagtat tttgacaaca ggactctaca gttttatctt 2520
   tttagtgtgc atgtgttctc cttttttttt gcaaatagct tcacctatat aatacttcat 2580
   ccattttatt agtacatcca tttagggttt agggttaatg gtttttatag actaattttt 2640
   ttagtacatc tattttattc tattttagcc tctaaattaa gaaaactaaa actctatttt 2700
   agttttttta tttaatagtt tagatataaa atagaataaa ataaagtgac taaaaattaa 2760
   acaaataccc tttaagaaat taaaaaaact aaggaaacat ttttcttgtt tcgagtagat 2820
   aatgccagcc tgttaaacgc cgtcgacgag tctaacggac accaaccagc gaaccagcag 2880
   cgtcgcgtcg ggccaagcga agcagacggc acggcatctc tgtcgctgcc tctggacccc 2940
   tctcgagagt tccgctccac cgttggactt gctccgctgt cggcatccag aaattgcgtg 3000
   gcggagcggc agacgtgagc cggcacggca ggcggcctcc tcctcctctc acggcaccgg 3060
   cagctacggg ggattccttt cccaccgctc cttcgctttc ccttcctcgc ccgccgtaat 3120
   aaatagacac cccctccaca ccctctttcc ccaacctcgt gttgttcgga gcgcacacac 3180
   acacaaccag atctccccca aatccacccg tcggcacctc cgcttcaagg tacgccgctc 3240
   gtcctccccc cccccccccc tctctacctt ctctagatcg gcgttccggt ccatgcatgg 3300
   ttagggcccg gtagttctac ttctgttcat gtttgtgtta gatccgtgtt tgtgttagat 3360
   ccgtgctgct agcgttcgta cacggatgcg acctgtacgt cagacacgtt ctgattgcta 3420
   acttgccagt gtttctcttt ggggaatcct gggatggctc tagccgttcc gcagacggga 3480
   tcgatttcat gatttttttt gtttcgttgc atagggtttg gtttgccctt ttcctttatt 3540
   tcaatatatg ccgtgcactt gtttgtcggg tcatcttttc atgctttttt ttgtcttggt 3600
   tgtgatgatg tggtctggtt gggcggtcgt tctagatcgg agtagaattc tgtttcaaac 3660
   tacctggtgg atttattaat tttggatctg tatgtgtgtg ccatacatat tcatagttac 3720
   gaattgaaga tgatggatgg aaatatcgat ctaggatagg tatacatgtt gatgcgggtt 3780
   ttactgatgc atatacagag atgctttttg ttcgcttggt tgtgatgatg tggtgtggtt 3840
   gggcggtcgt tcattcgttc tagatcggag tagaatactg tttcaaacta cctggtgtat 3900
   ttattaattt tggaactgta tgtgtgtgtc atacatcttc atagttacga gtttaagatg 3960
   gatggaaata tcgatctagg ataggtatac atgttgatgt gggttttact gatgcatata 4020
   catgatggca tatgcagcat ctattcatat gctctaacct tgagtaccta tctattataa 4080
   taaacaagta tgttttataa ttatttcgat cttgatatac ttggatgatg gcatatgcag 4140
   cagctatatg tggatttttt tagccctgcc ttcatacgct atttatttgc ttggtactgt 4200
   ttcttttgtc gatgctcacc ctgttgtttg gtgttacttc tgcagggtac ccccggggtc 4260
   gaccatggct catgctgccc tcagccctct ctcccaacgc tttgagagaa tagctgtcca 4320
   gccactcact ggtgtccttg gtgctgagat cactggagtg gacttgaggg aaccacttga 4380
   tgacagcacc tggaatgaga tattggatgc cttccacact taccaagtca tctactttcc 4440
   tggccaagca atcaccaatg agcagcacat tgcattctca agaaggtttg gaccagttga 4500
   tccagtgcct cttctcaaga gcattgaagg ctatccagag gttcagatga tccgcagaga 4560
   agccaatgag tctggaaggg tgattggtga tgactggcac acagactcca ctttccttga 4620
   tgcacctcca gctgctgttg tgatgagggc catagatgtt cctgagcatg gcggagacac 4680
   tgggttcctt tcaatgtaca cagcttggga gaccttgtct ccaaccatgc aagccaccat 4740
   cgaagggctc aacgttgtgc actctgccac acgtgtgttc ggttccctct accaagcaca 4800
   gaaccgtcgc ttcagcaaca cctcagtcaa ggtgatggat gttgatgctg gtgacagaga 4860
   gacagtccat cccttggttg tgactcatcc tggctctgga aggaaaggcc tttatgtgaa 4920
   tcaagtctac tgtcagagaa ttgagggcat gacagatgca gaatcaaagc cattgcttca 4980
   gttcctctat gagcatgcca ccagatttga cttcacttgc cgtgtgaggt ggaagaaaga 5040
   ccaagtcctt gtctgggaca acttgtgcac catgcaccgt gctgttcctg actatgctgg 5100
   caagttcaga tacttgactc gcaccacagt tggtggagtt aggcctgccc gctgagtagt 5160
   tagcttaatc acctagagct cgtttaaact gagggcactg aagtcgcttg acgtgctgaa 5220
   ttgtttgtga tgttggtggc gtattttgtt taaataagta agcatggctg tgattttatc 5280
   atatgatcga tctttggggt tttatttaac acattgtaaa atgtgtatct attaataact 5340
   caatgtataa gatgtgttca ttcttcggtt gccatagatc tgcttatttg acctgtgatg 5400
   ttttgactcc aaaaaccaaa atcacaactc aataaactca tggaatatgt ccacctgttt 5460
   cttgaagagt tcatctacca ttccagttgg catttatcag tgttgcagcg gcgctgtgct 5520
   ttgtaacata acaattgtta cggcatatat ccaatagcgg ccggcctcct gcagggttta 5580
   aacttgccgt ggcctatttt cagaagaagt tcccaatagt agtccaaaat ttttgtaacg 5640
   aagggagcat aatagttaca tgcaaaggaa aactgccatt ctttagaggg gatgcttgtt 5700
   taagaacaaa aaatatatca ctttcttttg ttccaagtca ttgcgtattt ttttaaaaat 5760
   atttgttcct tcgtatattt cgagcttcaa tcactttatg gttctttgta ttctggcttt 5820
   gctgtaaatc gtagctaacc ttcttcctag cagaaattat taatacttgg gatatttttt 5880
   tagaatcaag taaattacat attaccacca catcgagctg cttttaaatt catattacag 5940
   ccatataggc ttgattcatt ttgcaaaatt tccaggatat tgacaacgtt aacttaataa 6000
   tatcttgaaa tattaaagct attatgatta ggggtgcaaa tggaccgagt tggttcggtt 6060
   tatatcaaaa tcaaaccaaa ccaactatat cggtttggat tggttcggtt ttgccgggtt 6120
   ttcagcattt tctggttttt tttttgttag atgaatatta ttttaatctt actttgtcaa 6180
   atttttgata agtaaatata tgtgttagta aaaattaatt ttttttacaa acatatgatc 6240
   tattaaaata ttcttatagg agaattttct taataacaca tgatatttat ttattttagt 6300
   cgtttgacta atttttcgtt gatgtacact ttcaaagtta accaaattta gtaattaagt 6360
   ataaaaatca atatgatacc taaataatga tatgttctat ttaattttaa attatcgaaa 6420
   tttcacttca aattcgaaaa agatatataa gaattttgat agattttgac atatgaatat 6480
   ggaagaacaa agagattgac gcattttagt aacacttgat aagaaagtga tcgtacaacc 6540
   aattatttaa agttaataaa aatggagcac ttcatattta acgaaatatt acatgccaga 6600
   agagtcgcaa atatttctag atatttttta aagaaaattc tataaaaagt cttaaaggca 6660
   tatatataaa aactatatat ttatattttt tacccaaaag caccgcaagg ggtagccctg 6720
   ggtgtgcgga cggactctaa acaccgacag ctggcgcgcc aggtaggggg tgtgtctttg 6780
   atctgagcta gctcaatgac cattacctcc aaatgcaaga tcgcccttcg ccccgggact 6840
   atgttttgct ttggaaccat ctcatccata gcagatgaag agggaactct gcaccgcata 6900
   gcagatctat tggagaagaa gctttcctca gaaatctcga ggggagccag ggcagaacag 6960
   cgggtggcac catcacccgc acctcaagcg aagatgacct cttacaaacc gaaagtcggg 7020
   agctcaccta cccgaaaaac tccgctgtcc acttcgccca caaaggagtg gacacggatt 7080
   actcgaaaga aggaagcgag tgtcccgagt caggggacgg gaacacgcca agccatcttt 7140
   ccgacgcctt cgccctcaaa tgaggatgga aagaagagcg ccatcgcgct ggctcctttc 7200
   taccccgacg tcctcttcat cagggggaga ttggagttag cacccgtctt caacgatgag 7260
   ccaaccatgc aaggggaaga gcctccccag cgtgaggcgc gacgacggag gaatagaagc 7320
   cagaacgtgc ggcgacatca cgaggctggg gaacgggatc cggcgcaacc cgtatcccgg 7380
   gacgaagctt tagaagtagg aaaaactccc gacgagtggg tacaccgaga aaggcggaac 7440
   tctcgccgcc gtgatcgccg acaagcttag gaccgagaac gagagcaagc cgagcaaggt 7500
   gcaaggctgc gccgagagaa tgctctcttt gctcggaacc tgtaccccga cttcgctcgt 7560
   gcaatgaaca cgccgagtga agtcggaggg gtactggccc agatagctga cggcctcccg 7620
   cgaaccctag acacggaagg ctaccggcgg ctgcttactc gagcagttaa tcaccttcta 7680
   cccatcacta atcctccaag cgacctacgc catgccatca acagccggcg agacacgcgg 7740
   agctccatca acgcttcgcg cgaccgatga cacgaaagtg agatagggaa ccgagaggag 7800
   tatgtccgag atcatgccat cctggcatga agtcatgcca cccgagctga gtcggttgcg 7860
   gcctcgacca gtgtcccgtt ccagggacga tcaagatgac acacaactgg ctcccctcct 7920
   tgggaccgac ctcacgaacg ccgacatgaa gacacgtgcg gagtcttcgc acttactccg 7980
   tgtctccggg ccatccagtg gcccctaact tcaaggtctc caacgtcagc aagtatgagc 8040
   gcaagcagga cctgggtggc tggttagcca tctacacgat tgtcacatgg gccgccggag 8100
   cgacggagga cgtgatgaca gtgtattttc ccattgtcct agggcaagac gcaatgcagt 8160
   ggctccgaca tctaccccaa cattgcatag acaattggag cgacttcagt tggtgcttca 8220
   tcgccaactt ccagtccctc tttgacaagc cggcgcagcc atgggaccta aaatccattg 8280
   ggcatcaggg cgatgaaacg ctccggttgt acctcaagag gttttagacc atgaggaacc 8340
   acacccccga agtcgccgag gcgggggtga ttgaagactt ctaccgagga tccaatgact 8400
   cggctttcgt ccgagccata ctccagaaaa gcgtcggcca cctccgaaca cttgttccgg 8460
   gaggcagacc tctacatcac cacggattaa cgggcccagg acctcatcgg aggcacgaaa 8520
   gccgcgccac acgcgccacg gtgtgacacg aaccagc 8557
<210> 2
   <211> 23
   <212> DNA
   <213> Artificial Sequence
<220>
   <223> forward event primer
<400> 2
   attctggctt tgctgtaaat cgt 23
<210> 3
   <211> 24
   <212> DNA
   <213> Artificial Sequence
<220>
   <223> event reverse primer
<400> 3
   ttacaatcaa cagcaccgta cctt 24
<210> 4
   <211> 19
   <212> DNA
   <213> Artificial Sequence
<220>
   <223> event probe
<400> 4
   ctaaccttca ttgtattcc 19
<210> 5
   <211> 18
   <212> DNA
   <213> Artificial Sequence
<220>
   <223> reference forward primer
<400> 5
   tggcggacga cgacttgt 18
<210> 6
   <211> 19
   <212> DNA
   <213> Artificial Sequence
<220>
   <223> reference reverse primer
<400> 6
   aaagtttgga ggctgccgt 19
<210> 7
   <211> 26
   <212> DNA
   <213> Artificial Sequence
<220>
   <223> reference probe
<400> 7
   cgagcagacc gccgtgtact tctacc 26
<210> 8
   <211> 73
   <212> DNA
   <213> Artificial Sequence
<220>
   <223> event amplicon
<400> 8
   ttacaatcaa cagcaccgta ccttgaagcg gaatacaatg aaggttagct acgatttaca 60
   gcaaagccag aat 73
<210> 9
   <211> 79
   <212> DNA
   <213> Artificial Sequence
<220>
   <223> reference amplicon
<400> 9
   tggcggacga cgacttgtcc gagcagaccg ccgtgtactt ctacctgctc aagggcacgg 60
   acggcagcct ccaaacttt 79